# EUROPEAN PATENT SPECIFICATION

(11) **EP 1 397 998 B1**
(45) Date of publication and mention of the grant of the patent: **03.12.2008**
(21) Application number: 02710878.6
(22) Date of filing: 30.01.2002
(51) Int. Cl.: A61B 17/08

(54) **GASTRIC CLAMP FOR PERFORMING VERTICAL BAND GASTROPLASTY AND A GASTRIC BYPASS WITH LESSER CURVATURE**
MAGENKLEMME FÜR DIE DURCHFÜHRUNG EINER VERTIKALBAND-GASTROPLASTIE UND MAGENBYPASS MIT GERINGERER KRÜMMUNG
PINCE GASTRIQUE DESTINEE A LA REALISATION D'UNE GASTROPLASTIE EN BANDE VERTICALE ET D'UNE DERIVATION GASTRIQUE SUR PETITE COURBURE

(30) Priority: 12.02.2001 ES 200100306
(43) Date of publication of application: 17.03.2004
(73) Proprietor: Molina Trigueros, Luis Miguel, 28009 Madrid (ES)
(72) Inventor: Molina Trigueros, Luis Miguel, 28009 Madrid (ES)
(74) Representative: Schouller, Jean-Philippe
(86) International application number: PCT/ES2002/000039
(87) International publication number: WO 2002/064041

(56) References cited:
- EP-A1- 0 519 704
- US-A- 4 328 805
- US-A- 4 558 699
- US-A- 4 803 985

## Description

### OBJECT OF THE INVENTION

This invention refers to an instrument which would permit the replacement of stapling in VBG and gastric by-pass to the lesser curvature (in patients operated for morbid obesity by means of these procedures) by a clamp which is able to efficaciously compress the anterior and posterior gastric wall, parallel to the lesser curvature, thus making the small gastric reservoir.

### BACKGROUND TO THE INVENTION

Morbid obesity is a serious health problem which affects over 4 million people in the United States. In Spain, it is calculated that 2% of the population is affected, a figure which is increasing, as it is in the rest of the world. Morbid obesity not only reduces life expectancy in subjects suffering from the disease itself, it also predisposes or increases their susceptibility to other conditions, such as diabetes mellitus, arterial hypertension, myocardial ischemia, Obstructive Sleep Apnea Syndrome (O.S.A.S.), osteoarticular pathology,...

The only efficacious treatment is Surgery, which is indicated when the Body Mass index (BMI) is above 40 (BMI = Body weight in kg/height in m²), or when the BMI is above 35 in the event of so-called concomitant comorbidities (hypertension, diabetes, myocardial ischemia, O.S.A.S., ...).

The most commonly used surgical treatments include vertical banded gastroplasty (VBG), which consists of making a small gastric reservoir which leads to early satiety in the patient following eating, thereby restricting the capacity to take in food and to store it in the stomach, eventually leading to notable weight loss.

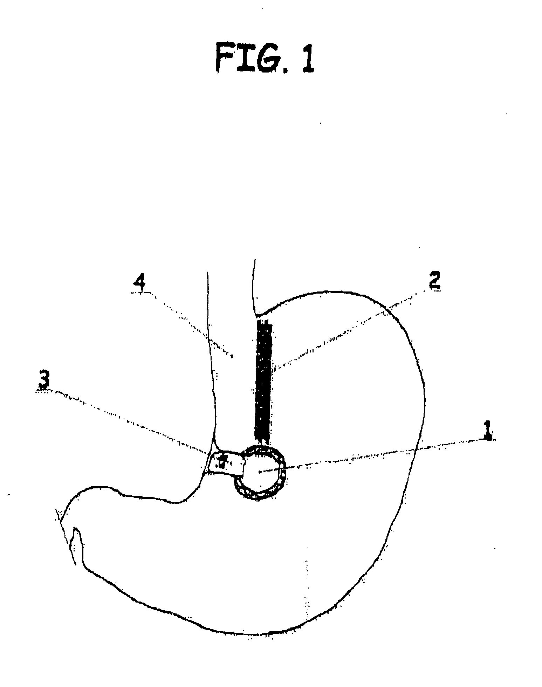

VBG is a simple and efficacious technique which in the 80s accounted for over 80% of bariatric surgery in the USA, although it presented a major problem which has yet to be solved, namely the shedding of staples, which conditions the production of gastric reservoir fistulae and eventually causes the patient to put on weight again. This has impacted the emergence of or search for other more complex surgical techniques (gastric by-pass, biliopancreatic diversion,...) which entall a greater surgical risk. VBG (Figure no. 1) consists of vertical stomach stapling (2) (performed using the bariatric stapler) parallel to the lesser curvature, following the making of a "gastric window" (1) 6-7 cm away from the oesophagogastric junction which, besides permitting the subsequent stapling of the viscera, permits the placement of a strip (3) (Marlex PTFE,...), thus notably reducing stomach-filling capacity, limiting it to the small gastric reservoir (4), ultimately making it difficult for food to go into the rest of the viscera.

Gastric stapling is carried out with the bariatric TA, which places 4 rows of metal staples. Some authors have used double stapling (8 rows of staples, partially superimposed), to avoid, as far as possible, the subsequent aforementioned gastric reservoir fistulae. This approach has reduced the incidence of fistulae from 15%-20% to 5%-10%, although it has not eliminated them completely. There are American Patents which have designed instruments to carry out gastroplasties:
- Hopkins, in 1982 (patent no. 4.458.681 USA), designed a clamp for horizontal gastroplasty with both plates of the centre area separated to allow food to pass to the distal stomach area. To my knowledge it has not been used in clinical practice. In any event, generally speaking horizontal gastroplasties have not been shown to be efficacious in Morbid Obesity.
- More recently, Bessier, in 1996 (patent no. 5.549.621 USA), designed a sophisticated instrument to carry out VBG which included two metal rods with penetrating spikes that emerge from the first clamp, until they adjust to the second "receiver" clamp, crossing the gastric wall completely. This instrument carries the output band of the VBG, To my knowledge it has not been used in clinical practice.

In both descriptions, the instrument does not have a curved external configuration or blunt edges. Nor is it lined with material that absorbs the pressure from the plates or rods.

Thus, the current alternative in Surgery to the instrument which is the object of this invention is the bariatric Stapler, which places metal staples and plates with spikes that cross the gastric wall completely (there is no clinical experience with this instrument).

US-A-4 558 699 discloses an apparatus for restricting the passage of food through a stomach. This apparatus, which is not adapted to vertical banded gastroplasty, includes two articulated jaws and a locking mechanism situated at opposite ends of the jaws.

### EXPLANATION OF THE INVENTION

The invention concerns a stomach clamp according to claim 1.

The gastric clamp for the performance of vertical banded gastroplasty is aimed at solving the problem posed by the shedding of staples, I.e, gastric reservoir fistulae in patients operated with this type of technique. The gastric "window" and the band at the very bottom of the gastric reservoir have exactly the same significance and are constructed in exactly the same way as the technical VBG technique.

The clamp which is the object of the invention consists of:
- Two (2) articulated plates 8.5 cm long and 10 mm wide, articulated at their bottom end by means of a hinge system with a stop which permits a rotation of 180° and with an automatic closure system at the top, which completes clamp closure, with the whole instrument compressing the anterior and posterior walls of the stomach with even pressure exerted on both plates.
- A "sealing" system which reinforces the automatic-closure system of the instrument preventing, in the event of a possible sudden pressure increase inside the gastric reservoir (produced by vomiting, which is not infrequent in VBG-operated patients), the opening of the automatic-closure system. This sealing system comprises a "female" part which embraces the top end of the clamp when it is closed.
- Both plates have two wide parts (one on each end of the instrument) on the right edge, both of which have two orifices which make it possible to fix the instrument to the gastric wall, by suture, thus preventing subsequent clamp movements.
- Since the clamp must remain in the patlent's body after surgery, a lightweight, light-alloy and highly resistant material, easily tolerated by the organism, is preferred for the design. Titanium complies with all these conditioning factors, although an inert plastic material may be used as the carcass or body of the instrument.
- Since the stomach is a soft viscera, whose consistency can in no way be likened to that of bone tissue, the whole clamp: 1) has blunt or curved edges all over the outside, barring the area where it comes into contact with the stomach (internal surface), being flat. This design would avoid the production of decubiti (pressure sores) in the actual
stomach viscera or adjacent structures, 2) is totally lined with material well tolerated by the organism and which, at the same time, duly cushions the pressure exerted by the clamp plates on the anterior and posterior gastric walls. The lining materials proposed are PTFE or Teflon.

This clamp may also be used occasionally in gastric by-pass to the lesser curvature, as can be seen in the corresponding drawing, and its width of 10 mm would allow passage of the instrument through a 12-mm trocar for positioning by means of laparoscopic surgery.

The advantages of the instrument which is the object of the invention are:
- A straightforward technique and easy-to-make gastric reservoir, both in VBG and in gastric by-pass to the lesser curvature.
- Replacement of barlatric stapler staples by the gastric clamp described, thus eliminating the possibility of staple shedding and gastric reservoir fistulae and therefore surgical failure.

### EXPLANATION OF THE DRAWINGS

For improved comprehension of all that is described in this report, technical drawings of the instrument and its location in the stomach are attached.
- Figure 1 represents a VBG as currently performed in bariatric surgery, for the purpose of comparing and understanding the distinguishing characteristics of this invention patent.

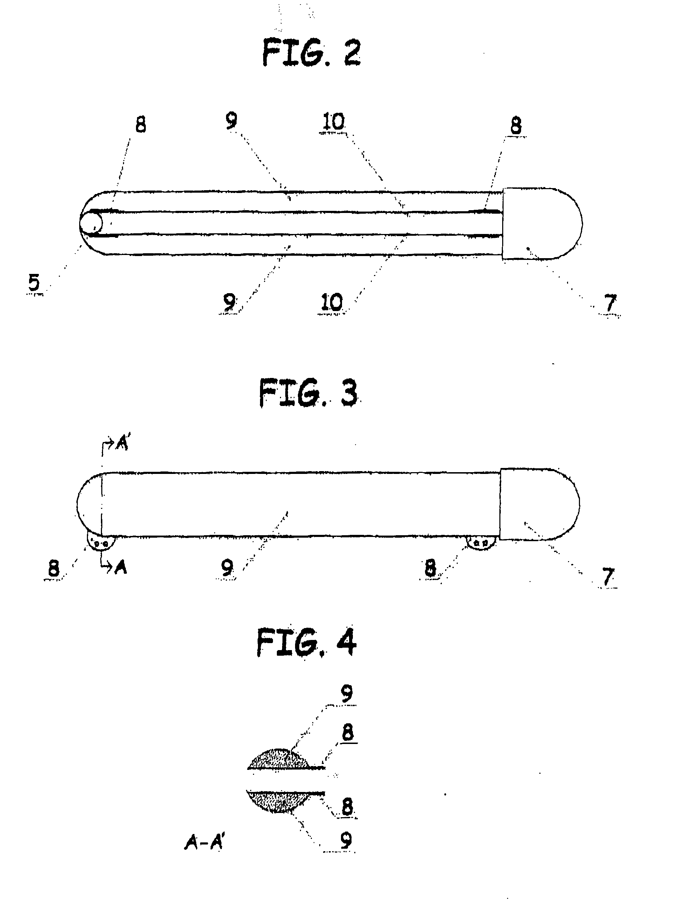

- Figure 2 shows a right elevation view of the clamp when closed
   Note: the left elevation view is similar to that of figure no. 2, the difference being that it does not have the wide parts for clamp fixation.
Figure no. 3 shows a plan view of the clamp.
Figure no. 4 shows a section of the clamp at A-A' level.

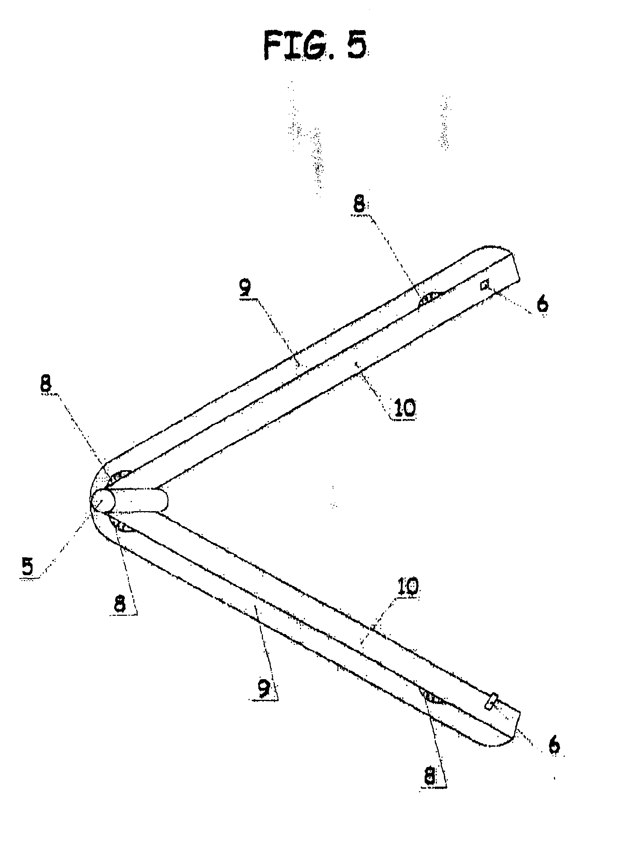

Figure no. 5 shows a right elevation view of the clamp opened at an angle of 45°, although rotation on the axis should reach 180°, as described previously.
Figure no. 6 shows a view of the open clamp being placed on the stomach. The diameter of the gastric reservoir is adjusted by means of a no. 32 Fouchet probe, as in conventional surgery.
Figure no. 7 shows a front view of the clamp in position and closed on the stomach, as well as the female reinforcement part, which will be inserted into the top end of the clamp.
Figure no, 7a shows the female sealing or reinforcement part positioned on the top end of the clamp.
Figure no. 8 shows a front view of a gastric by-pass to the lesser curvature (Torres-Oca technique) as performed in bariatric surgery, and figure no; 9 shows a view of the optional application of the instrument described, embracing the anterior and posterior walls of the stomach (in a more oblique position than in VBG), replacing gastric stapling.

### DETAILED DESCRIPTION OF THE INVENTION

The gastric clamp described for the creation of a VBG replaces gastric stapling, performed with bariatric TA - 2 -. The gastric window -1- and the PTFE strip -3- on the bottom end of the reservoir - 4 - retain exactly the same significance and are constructed in exactly the same way as the VBG technique currently used in conventional surgery for morbid obesity.
The gastric clamp which is the object of this invention comprises two plates which are 8.5 cm long and 10 mm wide. These dimensions are related, in the first case, to the length of the gastric reservoir, and in the second provide enough surface pressure to guarantee that the gastric reservoir will be impermeable to the rest of the viscera, while the area of gastric tissue that compresses the viscera will not be too extensive, in order to prevent ischemia or stomach vascularisation deficit, which could give rise to necrosis of the gastric wall. Previous experience has shown that pressure with clamps or rods offers greater advantage than staples, since the pressure exerted on the gastric wall that comes into contact with the rod is evenly distributed over its surface, whereas stapling exerts its pressure only on the areas where the viscera is penetrated by staples on the areas where the viscera is penetrated

Both plates are articulated on the bottom end by means of a hinge system - 5 - with a stop which permits a rotation of 180° on its axis (to achieve easy and comfortable placement as well as positioning by laparoscopic surgery through a 12mm trocar).

The top end of both plates presents an automatic closure system (clamp) -6-, which completes clamp closure, with the whole instrument compressing the anterior and posterior walls of the stomach with even pressure exerted between both plates.

The automatic closure system is reinforced with a sealing system -7- to prevent a sudden pressure surge inside the gastric reservoir from opening the clamp. This sealing system comprises a "female" part which embraces a 1,25-mm section of the top end of the clamp when the latter is closed.

The right edge (situated stomach-side opposite the small gastric reservoir) of the ends of both plates have two wide parts which present two orifices -8- that make it possible to fix the instrument to the gastric wall by means of non-reabsorbable suture stitches, both on the top edge and bottom part of the stomach next to the reservoir (fundus of stomach and gastric window made previously) thus preventing subsequent clamp movements.

As has already been expounded, the material used to make the instrument is lightweight, light-alloy, highly resistant and blocompatible (titanium or inter plastic material). The totality of the edges of the instrument have blunt or curved edges on the outside -9- , barring the area in contact with the stomach (Internal surface), being flat -10- . The whole instrument is lined with material which duly cushions the pressure exerted by the clamp plates on the anterior and posterior gastric walls, thus avoiding rejection by the organism. The lining materials proposed are PTFE or Teflon.

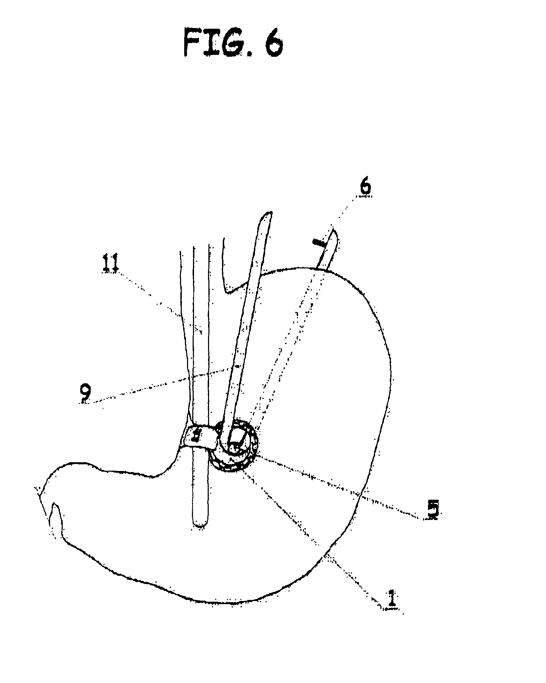

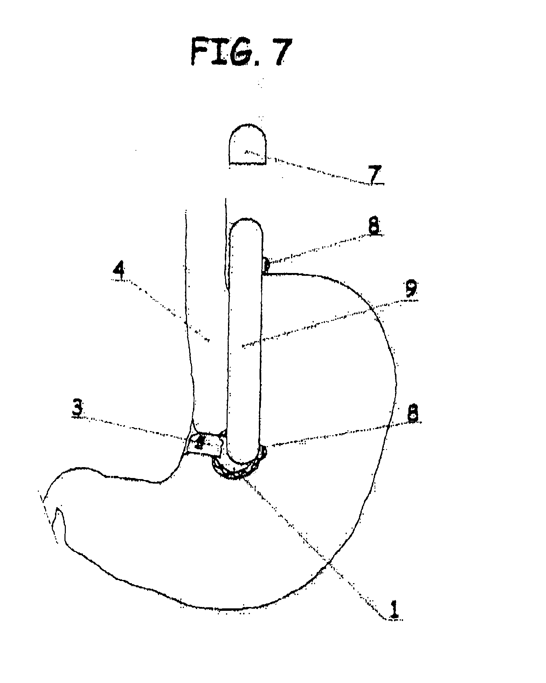

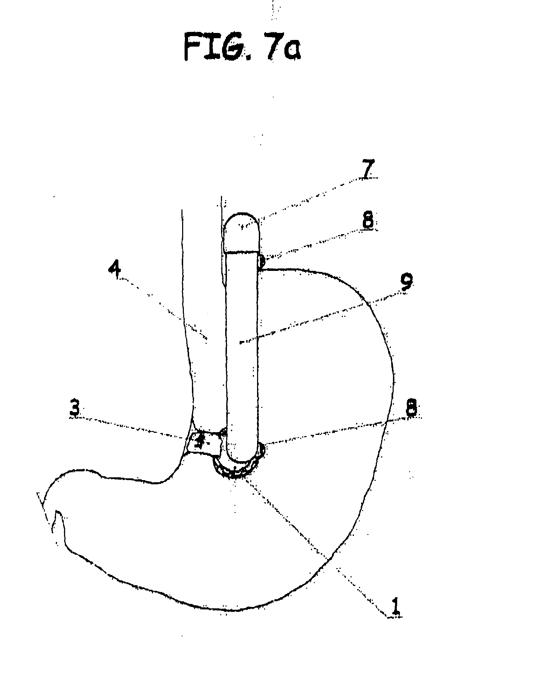

Surgical positioning in VBG is performed as follows - figures nos. 6, 7 and 7a. Once the gastric window has been made, by means of the no. 28 CEEA circular self-suture machine the rear plate is inserted behind the stomach until its top end emerges above the fundus of stomach, followed by the automatic closure of the instrument. Following closure, the female part of the sealing system is added. The instrument follows a vertical direction towards the oeseophagus and Hiss angle and the diameter of the gastric reservoir is adjusted by means of a no. 32 Fouchet probe -11 -, as in conventional surgery. Once the clamp has been positioned and closed on the stomach, it is fixed to the latter with suture stitches passed through the wide parts of the instrument -8 - on the fundus and gastric window, stomach-side opposite the reservoir. The Fouchet probe is withdrawn and the PTFE strip is placed on the distal end of the reservoir, as in conventional surgery.

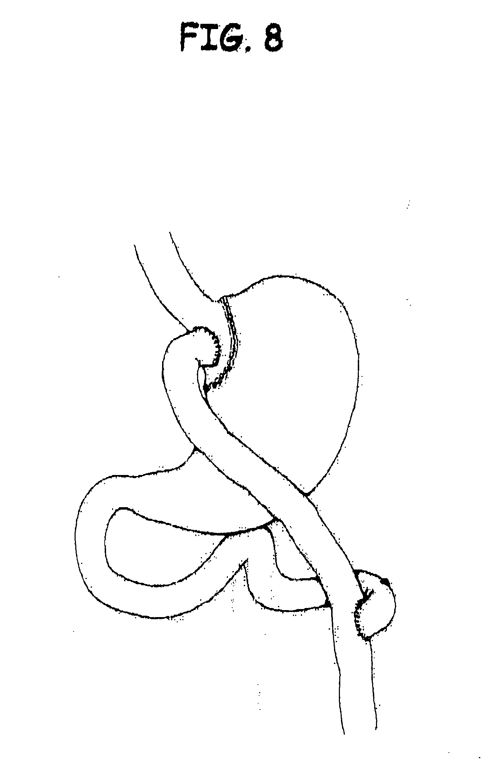

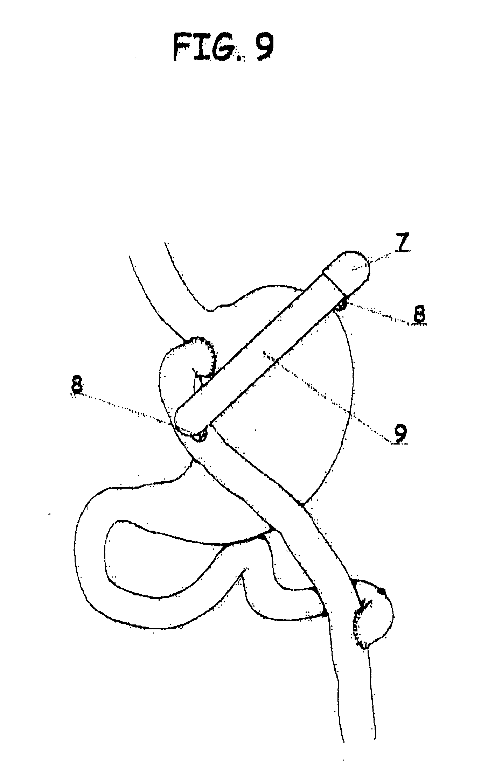

This clamp may also be used occasionally in gastric by-pass to the lesser curvature (figure no. 8), as can be seen in figure no. 9, while its width of 10 mm would permit the passage of the instrument through a 12-mm trocar for positioning by means of laparoscopic surgery,

## Claims

**1.** - Stomach clamp adapted for the performance of Vertical Banded Gastroplasty (VGB) and gastric by-pass to the lesser curvature, said clamp comprising
- a body including two articulated plates having each a flat internal surface (10) adapted to compress the anterior and posterior walls of a stomach with even pressure,
- an automatic closure system having cooperating parts (6) respectively provided on the free ends of said plates,
- a sealing system (7) which reinforces said closure system and which is adapted to prevent a pressure surge inside a gastric reservoir from opening the clamp
- a curved-line blunt-edge configuration
- a stomach fixation system (8) different from said closure and sealing systems and
- an integral lining adapted to cushion the pressure exerted by the clamp plates on the anterior and posterior gastric walls.

**2.** - Stomach clamp according to claim 1, **characterized in that** said cooperating parts (6) of said automatic closure system are located on the internal flat surface (10) of said plates.

**3.** - Stomach clamp according to one of claims 1 or 2, **characterized in that** both plates are articulated at their bottom ends by means of a hinge system (5) with a stop which allows a rotation of 180° on its axis, in such a manner that positioning of said clamp can be made by laparoscopic surgery.

**4.** - Stomach clamp according to one of claims 1 or 3, **characterized in that** the totality of the edges of the instrument have blunt or curved edges on the outside (9).

**5.** - Stomach clamp according to one of claims 1 to 4, **characterized in that** said cooperating parts (6) of said closure system are provided on ends of said plates opposite the ends by which they are articulated one on the other.

**6.** - Stomach clamp according to one of claims 1 to 5, **characterized in that** said sealing system includes a female part (7) which is independent of the rest of the clamp and which embraces a section of the open end of the clamp when the latter is closed.

**7.** - Stomach clamp according to one of claims 1 to 6, **characterized in that** said stomach fixation system comprises two wide parts (8) located on an edge of said plates, said edge being situated stomach-side opposite a small gastric reservoir when said clamp is installed on a stomach, said wide parts (8) having two orifices which allow the passage of suture stitches.

**8.** - Stomach clamp according to one of claims 1 to 7, **characterized in that** said clamp is made of titanium or an inert plastic material.

**9.** - Stomach clamp according to one of claims 1 to 8, **characterized in that** said lining is made of PTFE or TEFLON.

**10.** - Stomach clamp according to one of claims 1 to 9, **characterized in that** said plates are 8.5 cm long and 10 mm wide.

## Patentansprüche

1. Magenklemme, die für die Ausführung einer Vertical Banded Gastroplasty (VGB) und eines Magen-Bypasses an der kleinen Magenkurvatur angepasst ist, wobei die Klemme aufweist:
- einen Körper, der zwei gelenkig verbundene Platten enthält, von denen jede eine flache Innenfläche (10) aufweist, die dazu angepasst ist, die Vorder- und Hinterwand eines Magens mit gleichmäßigem Druck zusammenzudrücken;
- ein automatisches Schließsystem, das zusammenwirkende Teile (6) aufweist, die jeweils an den freien Enden der Platten vorhanden sind;
- ein Verschlusssystem (7), welches das Schließsystem verstärkt und das dazu angepasst ist, einen Druckstoß innerhalb eines Magenreservoirs daran zu hindern, die Klammer zu öffnen;
- eine gekrümmte, stumpfkantige Konfiguration;
- ein Magen-Fixiersystem (8), das von dem Schließ- und dem Verschlusssystem verschieden ist, und
- eine integrale Verkleidung, die dazu angepasst ist, den durch die beiden Klemmplatten auf die Vorder- und Hinterwand des Magens ausgeübten Druck abzuschwächen.

2. Magenklemme nach Anspruch 1, **dadurch gekennzeichnet, dass** sich die zusammenwirkenden Teile (6) des automatischem Schließsystems auf der flachen Innenfläche (10) der Platten befinden.

3. Magenklemme nach Anspruch 1 oder 2, **dadurch gekennzeichnet, dass** die beiden Platten an ihren unteren Enden mittels eines einen Anschlag aufweisenden Drehgelenksystems (5), das eine 180°-Drehung um seine Achse zulässt, derart angelenkt sind, dass das Positionieren der Klemme mittels laparoskopischer Operation durchgeführt werden kann.

4. Magenklemme nach Anspruch 1 oder 3, **dadurch gekennzeichnet, dass** alle Kanten des Instruments stumpfe oder gekrümmte Kanten auf der Außenseite (9) aufweisen.

5. Magenklemme nach einem der Ansprüche 1-4, **dadurch gekennzeichnet, dass** die zusammenwirkenden Teile (6) des Schließsystems an den Plattenenden vorhanden sind, die den Enden, an denen sie gelenkig miteinander verbunden sind, gegenüber liegen.

6. Magenklemme nach einem der Ansprüche 1-5, **dadurch gekennzeichnet, dass** das Verschlusssystem einen weiblichen Teil (7) aufweist, der von dem restlichen Teil der Klemme unabhängig ist und der einen Teilabschnitt des offenen Endes der Klemme umgreift, wenn letztere geschlossen ist.

7. Magenklemme nach einem der Ansprüche 1-6, **dadurch gekennzeichnet, dass** das Magen-Fixiersystem zwei breite Teile (8) aufweist, die sich an einer Kante der Platten befinden, wobei die Kante auf der Magenseite liegt, die dem kleinen Magenreservoir abgewandt ist, wenn die Klemme an einem Magen angebracht ist, und die breiten Teile (8) zwei Öffnungen aufweisen, die den Durchgang von Nahtstichen zulassen.

8. Magenklemme nach einem der Ansprüche 1-7, **dadurch gekennzeichnet, dass** die Klemme aus Titan oder einem inerten Kunststoffmaterial besteht.

9. Magenklemme nach einem der Ansprüche 1-8, **dadurch gekennzeichnet, dass** die Verkleidung aus PTFE oder TEFLON besteht.

10. Magenklemme nach einem der Ansprüche 1-9, **dadurch gekennzeichnet, dass** die Platten 8,5 cm lang und 10 mm breit sind.

## Revendications

1. Pince stomacale adaptée pour la réalisation d'une gastroplastie verticale (GV) ou d'un court-circuit gastrique vers la petite courbure de l'estomac, ladite pince comprenant :
- un corps incluant deux plaques articulées ayant chacune une surface interne plate (10) adaptée pour comprimer les parois antérieure et postérieure d'un estomac en appliquant une pression égale,
- un système de fermeture automatique ayant des parties coopérantes (6) prévues respectivement sur les extrémités libres desdites plaques,
- un système de verrouillage (7) qui renforce ledit système de fermeture et qui est adapté pour empêcher qu'un à-coup de pression à l'intérieur d'un réservoir gastrique n'ouvre la pince,
- une configuration de bord mousse à ligne incurvée,
- un système de fixation (8) à l'estomac différent desdits systèmes de fermeture et de verrouillage et
- un revêtement intégral adapté pour amortir la pression exercée par les plaques de la pince sur les parois gastriques antérieure et postérieure.

2. Pince stomacale selon la revendication 1, **caractérisée en ce que** lesdites parties coopérantes (6) dudit système de fermeture automatique se trouvent sur la surface plate interne (10) desdites plaques.

3. Pince stomacale selon l'une des revendications 1 ou 2, **caractérisée en ce que** les deux plaques sont articulées à leur extrémité inférieure au moyen d'un système de charnière (5) avec une butée qui permet une rotation de 180° sur son axe, de manière à ce que le positionnement de ladite pince puisse être réalisé par chirurgie laparoscopique.

4. Pince stomacale selon l'une des revendications 1 ou 3, **caractérisée en ce que** la totalité des bords de l'instrument ont des bords mousses ou incurvés, sur l'extérieur (9).

5. Pince stomacale selon l'une des revendications 1 à 4, **caractérisée en ce que** lesdites parties coopérantes (6) dudit système de fermeture sont prévues sur les extrémités desdites plaques à l'opposé des extrémités par lesquelles elles sont articulées l'une à l'autre.

6. Pince stomacale selon l'une quelconque des revendications 1 à 5, **caractérisée en ce que** ledit système de verrouillage inclut une partie femelle (7) qui est indépendante du reste de la pince et qui embrasse une partie de l'extrémité ouverte de la pince lorsque cette dernière est fermée.

7. Pince stomacale selon l'une des revendications 1 à 6, **caractérisée en ce que** ledit système de fixation stomacale comprend deux parties larges (8) situées sur un bord desdites plaques, ledit bord étant situé du côté de l'estomac à l'opposé d'un petit réservoir gastrique lorsque ladite pince est installée sur un estomac, lesdites parties larges (8) ayant deux orifices permettant le passage de points de suture.

8. Pince stomacale selon l'une des revendications 1 à 7, **caractérisée en ce que** ladite pince est fabriquée en titane ou en un matériau plastique inerte.

9. Pince stomacale selon l'une des revendications 1 à 8, **caractérisée en ce que** ledit revêtement est en PTFE ou TEFLON.

10. Pince stomacale selon l'une des revendications 1 à 9, **caractérisée en ce que** lesdites plaques ont une longueur de 8,5 cm et une largeur de 10 mm.
